Europäisches Patentamt

European Patent Office

Office européen des brevets

(11) Publication number: **0 425 119 A1**

# EUROPEAN PATENT APPLICATION

(21) Application number: 90310991.6

(22) Date of filing: 08.10.90

(51) Int. Cl.⁵: **G01N 27/12, G01N 33/00**

(30) Priority: 24.10.89 GB 8923924

(43) Date of publication of application:
02.05.91 Bulletin 91/18

(84) Designated Contracting States:
**BE DE FR GB IT NL**

(71) Applicant: **British Gas plc**
**Rivermill House 152 Grosvenor Road**
**London SW1V 3JL(GB)**

(72) Inventor: **Chadwick, Alan Vernham,**
**'Melrose' 34, Highfield Close**
**Canterbury, Kent CT2 9DX(GB)**
Inventor: **Wilson, Alan**
**29 Suffold Road**
**Canterbury, Kent CT1 1SA(GB)**
Inventor: **Wright, John Dalton,**
**'Robinbrake' Redcot Lane, Sturry**
**Canterbury, Kent CT2 0ES(GB)**

(74) Representative: **Morgan, David James**
**British Gas plc Patents, Licensing &**
**Commercial Dev. 326 High Holborn**
**London WC1V 7PT(GB)**

(54) Determining concentration of pollutant gas in atmosphere.

(57) The invention relates to apparatus 1 and method for determining the concentration of a pollutant gas such as NOx or CO/CH4 in a gaseous atmosphere such as ambient air, there being a pollutant gas sensor 2 on which the gas is adsorbed so that a current signal of the sensor 2 is altered and can be converted to a concentration determination the sensor 2 then being desorbed by heating to a higher (200° C) of the two predetermined temperature so it reverts to its initial current signal before being reset to the first temperature for repeating the determination. During desorption of adsorbed NOx from the sensor at the higher temperature, ambient air is cleaned by passing through a by-pass filter 15 before it enters a chamber 10 housing the sensor 2.

# DETERMINING CONCENTRATION OF POLLUTANT GAS IN AN ATMOSPHERE

The invention relates to determining concentration of pollutant gas in an atmosphere.

It is often necessary to determine the concentration of a particular gas in say ambient air, the particular gas often being noxious and comprising a pollutant. If the concentration of the gas rises above a particular level, the source can be adjusted to reduce the concentration to environmentally acceptable levels. Prior apparatus and methods of pollutant concentration determination have been proposed, but such are often not applicable for repeated sampling of a flow of gas and nor do they provide accuracy if they are repeatable. The results obtained are often inconsistent.

It is accordingly an object of the invention to seek to mitigate these disadvantages.

According to one aspect of the invention there is provided a method for determining the concentration of a pollutant gas in a carrier gas contaminated with the pollutant gas, the method comprising the steps of passing the carrier gas in the form in which it is contaminated with the pollutant over a sensor adapted to adsorb the pollutant gas for a first period, measuring the quantity of the pollutant gas so absorbed at the end of the first period, and then passing the carrier gas in a form in which it is substantially free of the pollutant gas over the sensor for a second period, so as to desorb the pollutant gas from the sensor into the carrier gas.

The length of the second period may be dependent upon the quantity of pollutant gas absorbed during the first period. This provides for accurate measurement.

The method may provide that during the first period the temperature of the sensor may be held at a first level and during the second period the temperature of the sensor may be held at a second level which may be higher than the first level.

The first temperature may be 150° C and the second temperature may be 200° C.

The pollutant gas may be removed from the carrier gas by passing the carrier gas through a filter prior to the carrier gas contacting the sensor.

The pollutant gas may comprise one or more of the following namely an oxide or oxides of nitrogen, carbon monoxide or methane.

In this method, the carrier gas is preferably air, suitably atmospheric air.

According to a second aspect of the invention there is provided apparatus for determining the concentration of a pollutant gas in a carrier gas contaminated with the pollutant gas, the apparatus comprising a sensor adapted to absorb the pollutant gas, a chamber containing the sensor, the chamber having an inlet to receive the carrier gas and an outlet to discharge the carrier gas, means for causing flow through the chamber of the carrier gas from the inlet to the outlet so that the carrier gas passes over the sensor, means for measuring the quantity of pollutant gas absorbed on the sensor, and means selectively operable to cause the carrier gas to reach the sensor either in the form in which the carrier gas is contaminated with the pollutant gas or in a form in which the carrier gas is substantially free of the pollutant gas.

The seletively operable means may comprise a by-pass connected between the inlet and the chamber, a filter located in the by-pass for filtering out the pollutant gas from the carrier gas and a valve operable to connect the inlet either directly to the chamber or alternatively by way of the by-pass.

The apparatus may include means to control the selectively operable means so that the carrier gas may be caused to reach the chamber in its contaminated form for a first period and in its pollutant gas free form for a second period.

There may also be means provided to control the temperature of the sensor so that during the first period the temperature may be held at a first level and during the second period the temperature may be held at a second level which is higher than the first level.

An apparatus and a method for determining the concentration of a particular gas are hereinafter described, by way of example, with reference to the accompanying drawings.

Fig. 1 is a block diagram of apparatus for determining the concentration of nitrogen oxides (NOx) or CO/CH4 is a gas stream;

Fig.2 is a flow chart showing operational steps in using the apparatus or system of Fig 1;

Fig 3 is a schematic representation of analogue and digital signals resulting from adsorption of NOx in the apparatus or system of Fig 1; and

Fig 4 is a diagram of a circuit used in the apparatus of Fig 1.

Referring to the drawings there is shown apparatus 1 for carrying out a method for determining the concentration of a pollutant gas, in this case NOx in an atmosphere of ambient air, comprising a sensor 2 adapted to provide a change of electrical characteristic, in this case current, the presence of the NOx at one of two determined temperatures, means 3 to expose the sensor 2 to the air, means 4 to determine the change of electrical characteristic of the sensor from an original or base value of the characteristic, means 5, 6 to convert the electrical characteristic to a measure of concentration of the NOx in the air, and means 7 to control the temperature of the sensor 2 at the one predetermined value and to change the temperature to the second predetermined value at which the sensor 2 is prepared for reversion to the original electrical characteristic. Between determination or measurement cycles, clean ambient or atmospheric air is, in the apparatus 1 operated in a manner embodying the method of the invention, passed over the sensor 2 to desorb adsorbed NOx.

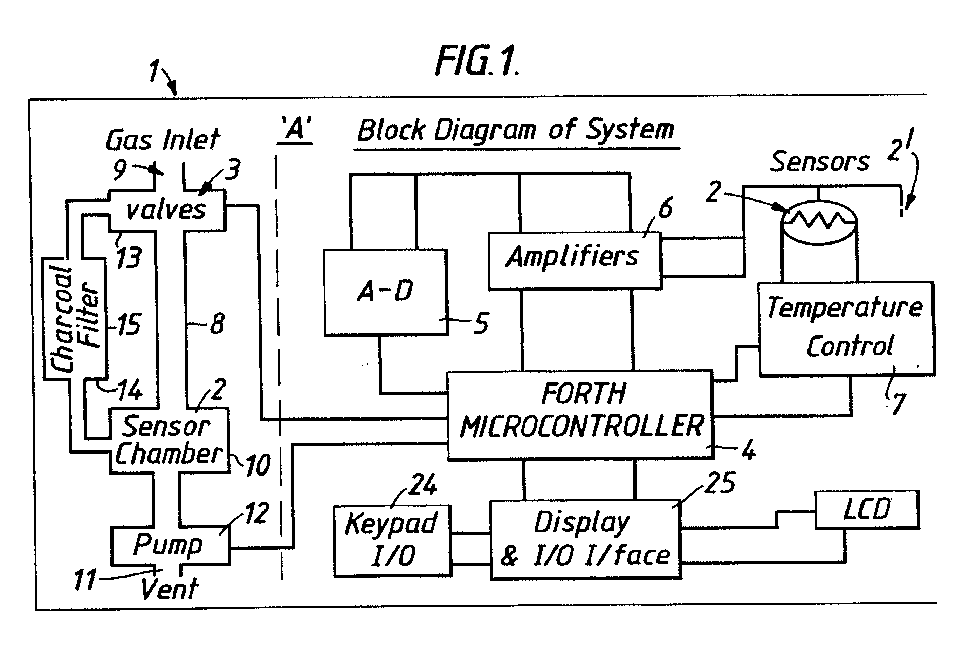

The apparatus 1 (Fig. 1) comprises a body 8 through which the air passes from an inlet 9 through a chamber 10 housing a sensor 2, or a plurality of sensors 2, 2' etc, to an outlet or vent 11 via a pump 12. The means 3 is in the form of a valve in a chamber 13 downstream of the inlet 9 and upstream of the chamber 10. There is also a passage or bypass 14 containing a filter device in the form of a charcoal filter 15 intermediate the valve 3 and the sensor chamber 10 so that the air can be passed on suitable operation of the valve 3 through the filter 15 before passing to the sensor(s) in the chamber 10,so initially removing substantially all the NOx during desorption from the sensor(s) 2, 2' etc.

The valve 3 is a three-way type solenoid valve controlled by the means 4 which is a microprocessor. During measurement phases the inlet 9 is connected directly to the sensor chamber 2 and the by-pass 14 is closed. During cleaning (desorption) phases the inlet 9 is connected to the chamber 10 solely by way of the by-pass 14.

The or each sensor 2 for sensing NOx is in the form of a thin sublimed film of an organic semiconductor material, for example lead phthalocyanine (PbPc). Where the or each sensor 2 is for determining CO/CH4 mixture, it or they comprise single crystals of metallic oxides, usually tin oxides (SnO2).

The sensor(s) 2 is or are heated to one or the other of two predetermined temperature values using the means 7 which includes a circuit incorporating feedback to maintain a set resistance of a heater (not shown) embedded in an alumina substrate (also not shown). The respective NOx and CO/CH4 sensors are maintained at about 150°C and at this temperature adsorb selectively the gases the concentration of which they are used to determine in the ambient air. By operating the apparatus 1 or system at 150°C at the first or one predetermined temperature, reproducible behaviour on exposure NOx in varying relative humidity is achieved. Using a plurality of sensors comprising a combination of NOx and CO/CH4 sensors, selectivity of determination of a particular gas can be achieved as the SnO2 (CO/CH4) sensor 2 does not respond in the same way as the PbPc (NOx) sensor 2.

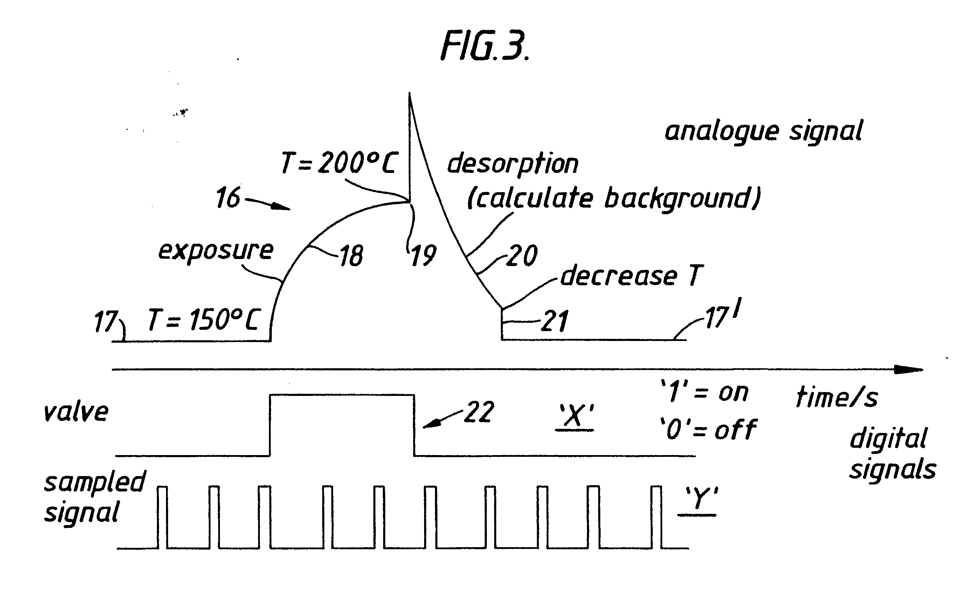

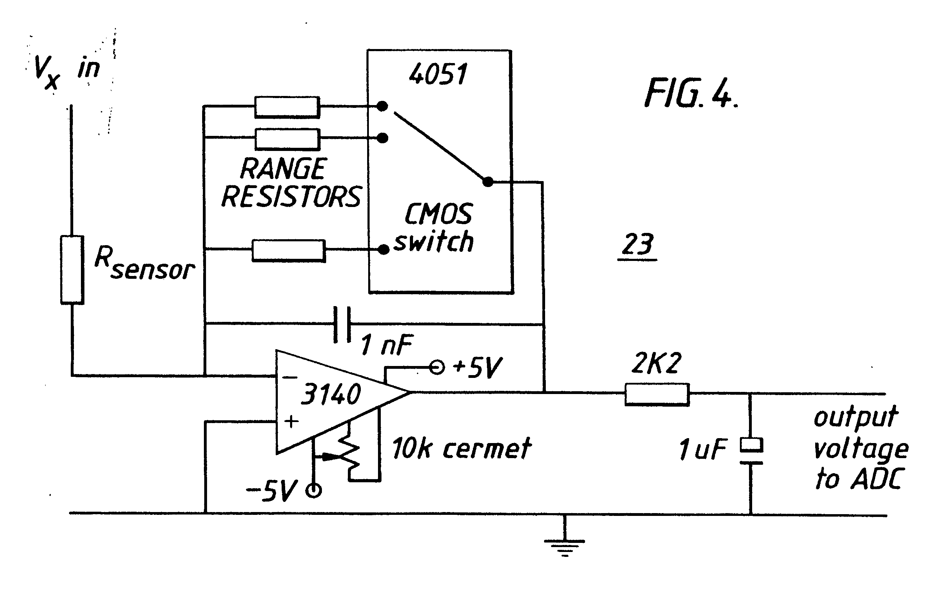

Whichever pollutant gas is being determined using the relevant sensor, for example the NOx sensor, NOx is adsorbed onto the surface of the sensor 2 at 150°C thereby producing a change in electrical characteristic from a base level at 150°C. The electrical characteristic which is monitored is the current. The sensor 2 has a constant voltage applied to it and when the adsorption takes place, the resistance of the sensor alters so as inevitably to change the current according to Ohm's law. The measured current is converted to a voltage, using a variable gain, high input impedance MOSFET operational amplifier 6 for reading by an analogue to digital converter 5, A-D (Fig 1), which can only read voltage. The analogue signal is shown in the top plot 16 in Fig 3, showing at 17 the sensor at 150°C, exposure at 18 to the NOx, raising to 200°C at 19 to desorb the NOx from the sensor 2 and to prepare the sensor for reversion at 20 to the original characteristic prior to decreasing the temperature at 21 to 150°C preparatory to a second or repeat determination of NOx at 17'. The operation of the valve(s) 3 is shown at 22 in Fig 4, the digital plot at "X" showing the opening of the valves 3 for exposure of the sensor 2 to the air. The digital plot of the sampled signal is shown at "Y" the lower plot in Fig 3.

In the method, ambient air including a pollutant such as NOx the concentration of which is to be determined, is passed to the apparatus 1 and the polluted gas is sampled at the inlet 9 using the valve 3 and the pump 12. The microprocessor 4 controls the three-way valve 3 so that the polluted air is either routed directly to the sensor(s) 2 during pollutant measurement, or via the charcoal filter 15, the air being cleaned by passing it through the by-pass filter 15 during the desorption cycle of the method and before passing to the sensor(s) 2, 2' etc. This removes substantially all the NOx, and allows cleaning of the filter without adversely affecting the sensor(s).

The temperature of the or each sensor 2 is kept constant at the first predetermined temperature (150°C) using the circuit A, Fig 1 and the sensor signal is amplified at 6 and converted to a digital signal at 5 for signal processing, as follows:-

The current signal from a sensor 2 is conditioned to ensure it lies within a range acceptable to the input stage of the means 5, 6 using an operational amplifier circuit 23, Fig 4 with the bias voltage set at such a value as to prevent the output exceeding the maximum input voltage for the ADC (usually + 5V)

Using a look-up table or calibration curve stored in ROM the microprocessor 4 calculates the gas (NO) concentration from the original current signal from the sensor 2. The apparatus is, it will be understood, calibrated by passing known concentration of a calibration gas (NOx, CO, CH4) over the sensor 2 and the signal reading monitored and compared with the expected value. In practice, on site testing of a gas for pollutant gas can be disrupt by a failed sensor 2 as replacing that sensor is time-consuming. On-site calibration would therefore be envisaged to be accomplished by measuring sensor response at a known low concentration, at a high concentration, and at say two intermediate levels, and generating an equation to fit the curve. This can be achieved interactively using a key pad 24 of the apparatus 1, so that re-calibration can be achieved quickly and easily. Thus in essence two parameters are stored to represent the calibration equation, namely the intercept and slope of a straight line.

After calculating the NOx concentration from the original current signal, further processing using the current of Fig 4, may be necessary if that signal is very low or very high, corresponding respectively to low and high NOx concentrations. The current of Fig 4 includes an autoranging amplifier adapted to measure current in the range μA to mA.

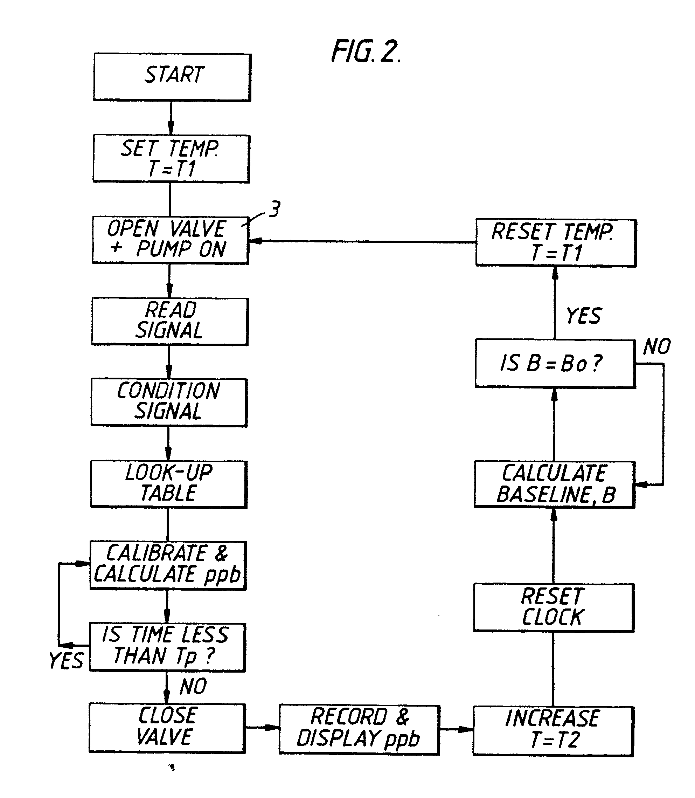

A comparison is then made between time of exposure (or pulse length) and pre-set exposure length (TP in Fig 2), and if the time is less than the time of exposure the preceding sequence of conditioning and calculation is repeated, otherwise the concentration of NOx in parts per billion (thousand million) (ppb) or parts per million (ppm) is recorded and displayed on the liquid crystal display. The time of exposure may, it will be understood, be altered by the apparatus 1 depending on the observed signal. In this way minimisation of water contamination and adverse chemical reaction on the surface of the or each sensor 2 is achieved. With regard to alteration of the time of exposure, at the two extremes of possible operation different problems are encountered, namely (a) when the NOx concentration is too low, the exposure time must be long to achieve necessary interaction and hence produce a measurable signal, and (b) when the NOx concentration is too high, a lengthy exposure to such high concentration produces a chemical reaction on the surface of the sensor 2, rather than straight forward adsorption, so limiting the lifetime and reliability of the sensor. One solution is to select an "optimum" time of exposure say 20 seconds based on empirical data derived from a wide rang of NOx concentrations likely to be encountered in practice. Alternatively, the apparatus 1 can be preprogrammed with upper and lower levels using an integral non-volatile memory, and then by comparing the measured NOx concentration with a stored value, and the unacceptable time of exposure can then be altered, say 20 seconds. This does not require any additional apparatus integer, the time of exposure or pulse length being set in software and hence readily changed.

After the NOx concentrations recorded and displayed at 25, the first temperature of 150° C is raised to the second predetermined value of 200° C to drive off the NOx from the sensor 2 so that it reverts to its initial current signal characteristic. The clock is reset.

The new signal is compared with the stored baseline BO, allowing for the change in temperature to 200° C and the apparatus 1 waits until the baseline BO is achieved again before allowing a new determination of NOx concentration to be made. At this juncture, the temperature is reset to 150° C and the apparatus 1 or system prompt is displayed to allow a new determination.

It will be understood that the apparatus 1 or system allows for storage in permanent memory of parameters such as the initial base-line and pulse lenght. An operator may also alter these values directly, and stored in an EEPROM. Other data such as calibration data may be input from the key pad 24.

It will be understood that while the method has been described for determining NOx concentration, a similar method may be used in CO/CH4 determination. Where the apparatus 1 includes a plurality of sensors, some of the NOx and some of the CO/CH4 variety, concentrations of these respective gases may be determined in one operation.

The apparatus and method may be utilised to determine pollutant gas concentration in air, exhaust gases or other gaseous atmospheres.

## Claims

1. A method for determining the concentration of a pollutant gas in a carrier gas contaminated with the pollutant gas, the method comprising the steps of passing the carrier gas in the form in which it is contaminated with the pollutant over a sensor adapted to adsorb the pollutant gas for a first period, measuring the quantity of the pollutant gas so absorbed at the end of the first period, and then passing the carrier gas in a form in which it is substantially free of the pollutant gas over the sensor for a second period, so as to desorb the pollutant gas from the sensor into the carrier gas.

2. A method as claimed in Claim 1 in which the length of the second period is dependent upon the quantity of pollutant gas absorbed during the first period.

3. A method as claimed in Claim 1 or Claim 2 in which during the first period the temperature of the sensor is held at a first level and during the second period the temperature of the sensor is held at a second level which may be higher than the first level.

4. A method as claimed in Claim 3 in which the first temperature is 150°C and the second temperature is 200°C.

5. A method as claimed in any of the proceding claims in which the pollutant gas is removed from the carrier gas by passing the carrier gas through a filter prior to the carrier gas contacting the sensor.

6. A method as claimed in any of the proceding claims in which the pollutant gas comprises one or more of the following namely an oxide or oxides of nitrogen, carbon monoxide or methane.

7. A method as claimed in any of the preceding claims in which the carrier gas is air.

8. A method as claimed in Claim 7 in which the air is atmospheric air.

9. A method substantially as hereinbefore described with reference to the drawings.

10. Apparatus for determining the concentration of a pollutant gas in a carrier gas contaminated with the pollutant gas, the apparatus comprising a sensor adapted to absorb the pollutant gas, a chamber containing the sensor, the chamber having an inlet to receive the carrier gas and an outlet to discharge the carrier gas, means for causing flow through the chamber of the carrier gas from the inlet to the outlet so that the carrier gas passes over the sensor, means for measuring the quantity of pollutant gas absorbed on the sensor, and means selectively operable to cause the carrier gas to reach the sensor either in the form in which the carrier gas is contaminated with the pollutant gas or in a form in which the carrier gas is substantially free of the pollutant gas.

11. Apparatus as claimed in Claim 10 in which the seletively operable means comprises a by-pass connected between the inlet and the chamber, a filter located in the by-pass for filtering out the pollutant gas from the carrier gas and a valve operable to connect the inlet either directly to the chamber or alternatively by way of the by-pass.

12. Apparatus as claimed in Claim 10 or Claim 11 in which means are provided to control the selectively operable means so that the carrier gas is caused to reach the chamber in its contaminated form for a first period and in its pollutant gas free form for a second period.

13. Apparatus as claimed in any of Claims 10 to 12 in which means are provided to control the temperature of the sensor so that during the first period the temperature may be held at a first level and during the second period the temperature may be held at a second level which is higher than the first level.

14. Apparatus substantially as hereinbefore defined with reference to the accompanying drawings.

# FIG.1.

Block Diagram of System

Gas Inlet · 'A' · Sensors

valves · Amplifiers · A-D · Temperature Control · FORTH MICROCONTROLLER · Charcoal Filter · Sensor Chamber · Pump · Vent · Keypad I/O · Display & I/O I/face · LCD

EP 0 425 119 A1

FIG. 2.

```
┌─────────────┐
│    START    │
└──────┬──────┘
       │
       ▼
┌─────────────┐                                    ┌─────────────┐
│  SET TEMP.  │                                    │ RESET TEMP. │
│   T = T1    │                                    │   T = T1    │
└──────┬──────┘                                    └──────┬──────┘
       │                3                                 ▲
       ▼                                                  │
┌─────────────┐◄──────────────────────────────────       │ YES
│ OPEN VALVE  │                                    ┌──────┴──────┐    NO
│  + PUMP ON  │                                    │  IS B = Bo? ├────┐
└──────┬──────┘                                    └──────▲──────┘    │
       │                                                  │           │
       ▼                                                  │           │
┌─────────────┐                                    ┌──────┴──────┐    │
│    READ     │                                    │  CALCULATE  │◄───┘
│   SIGNAL    │                                    │ BASELINE, B │
└──────┬──────┘                                    └──────▲──────┘
       │                                                  │
       ▼                                                  │
┌─────────────┐                                    ┌──────┴──────┐
│  CONDITION  │                                    │    RESET    │
│   SIGNAL    │                                    │    CLOCK    │
└──────┬──────┘                                    └──────▲──────┘
       │                                                  │
       ▼                                                  │
┌─────────────┐                                           │
│   LOOK-UP   │                                           │
│    TABLE    │                                           │
└──────┬──────┘                                           │
       │                                                  │
       ▼                                                  │
┌─────────────┐                                           │
│ CALIBRATE & │◄─┐                                        │
│CALCULATE ppb│  │                                        │
└──────┬──────┘  │                                        │
       │         │                                        │
       ▼         │                                        │
┌─────────────┐  │                                        │
│IS TIME LESS │  │ YES                                    │
│  THAN Tp ?  ├──┘                                        │
└──────┬──────┘                                           │
       │ NO                                               │
       ▼                                                  │
┌─────────────┐    ┌─────────────┐    ┌─────────────┐
│    CLOSE    │───►│  RECORD &   │───►│  INCREASE   │
│    VALVE    │    │ DISPLAY ppb │    │   T = T2    │
└─────────────┘    └─────────────┘    └─────────────┘
```

# FIG.3.

analogue signal

$T = 200°C$

desorption
(calculate background)

16 →

exposure

18    19    20

decrease T

17   $T = 150°C$      21      17'

valve      22    'X'    '1' = on    time/s

'0' = off    digital signals

sampled signal    'Y'

EP 0 425 119 A1

FIG. 4.

23

output voltage to ADC

2K2

1 uF

4051

CMOS switch

RANGE RESISTORS

10k cermet

+5V

1 nF

3140

−5V

$R_{sensor}$

$V_x$ in

9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | GB-A-1 479 925 (WESTFÄLISCHE BERGGEWERK-SCHAFTSKASSE) <br> * Page 2, lines 93-126; page 3, lines 103-118 * <br> — — — | 1,3-7 | G <br> 01 N 27/12 <br> G 01 N 33/00 |
| Y | GB-A-1 448 307 (NOHMI BOSAI KOGYO) <br> * Claim 1 * <br> — — — | 1,3-7 | |
| A | US-A-3 906 473 (H.D. LE VINE) <br> * Front page * <br> — — — | 1 | |
| A | EP-A-0 239 233 (WAKABAYASHI) <br> * Claim 1 * <br> — — — — — | 1 | |
| | | | TECHNICAL FIELDS SEARCHED (Int. Cl.5) |
| | | | G 01 N |

The present search report has been drawn up for all claims

| Place of search | Date of completion of search | Examiner |
|---|---|---|
| The Hague | 23 January 91 | DUCHATELLIER M.A. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document